# EUROPEAN PATENT SPECIFICATION

(11) **EP 0 558 747 B1**
(45) Date of publication and mention of the grant of the patent: **07.01.1998**
(21) Application number: 91912818.1
(22) Date of filing: 26.06.1991
(51) Int. Cl.: G01N 33/53, G01N 33/68, G01N 33/566

(54) **IMMUNOSORBENT FOR DIAGNOSIS OF EPILEPSY AND GROUP OF RISK**
IMMUNADSORBENS ZUR DIAGNOSE VON EPILEPSIE UND DER RISIKOGRUPPE
IMMUNOADSORBANT UTILISE DANS LE DIAGNOSTIC DE L'EPILEPSIE ET D'UN GROUPE A RISQUE

(43) Date of publication of application: 08.09.1993
(73) Proprietor: OBSCHESTVO S OGRANICHENNOI OTVETSTVENNOSTJU " DIES", Sankt-Peterburg, 196070 (RU)
(72) Inventor: DAMBINOVA, Svetlana Alexandrovna, St. Petersburg 197022 (SU); GORODINSKY, Alexandr Izarovich, St. Petersburg 192286 (SU); DEMINA, Marina Nikolaevna, St. Petersburg 197136 (SU)
(74) Representative: von Füner, Alexander, Dr.
(86) International application number: SU9100123
(87) International publication number: WO9300586

(56) References cited:
- BULLETIN OF EXPERIMENTAL BIOLOGY AND MEDICINE vol. 94, no. 12 , 1 December 1982 , NEW YORK, NY USA pages 1686 - 1688 S.A. DAMBINOVA ET AL 'Binding of 3H-L-glutamate with synaptic membranes isolated from the cerebral cortex and hippocampus of Krushinskii-Molodkina rats.' *

## Description

The present invention relates generally to medicine and more specifically to the immunosorbent for epilepsy and the risk group identification, finding application in the psychoneurological practice for the epilepsy diagnosis and the risk-group identification.

Epilepsy is a severe brain disease that affects about 1-4% of population. Nowadays its diagnosis is carried out on the basis of patients's clinical investigation which includes electrophysiological and X-ray techniques as well as other instrumentations. Such approach does not permit to evaluate unambiguously the brain excitatory pathways disturbances and related central nervous system disorders. Moreover the possible risk group identification is virtually excluded.

For the epilepsy diagnosis improvement the biochemical analysis of the patient's biological fluids for neurotransmitters (glutamate, gamma-aminobutyric acid, serotonin, dopamine and others) or their metabolites is usually used. However in a number of cases such analysis does not provide the expected results, not allowing unambiguous evaluation of specific metabolic disturbances in the brain, predisposition (risk group) identification, and the diagnosis on the early stages of the disease.

At the same time to find the reliable biochemical parameters valid for the objective estimation of epilepsy patients' condition is particularly important for the psycho-neurological clinics and dispensaries including those for children. The problem's solution is urgent for the epilepsy prophylactic measures and prediction especially in the professional aptitude test with a view to evaluate risk groups among personnel expected to operate under stressing conditions.

In a number of publications the presence of autoantibodies against brain antigens in the epileptic and other neurologic patients' blood was demonstrated. Total or partially fractionated brain tissue extracts, as well as several well-known brain-specific proteins, such as S-100 (Immunologia, V. 4, N 2, P. 75, 1974) were tried as antigens. The antigens mentioned above have no specificity in reference to any individual disease and cannot be used for the epilepsy diagnosis.

The herein-proposed immunosorbent for epilepsy and the risk group identification is substantially novel and has not so far been described in literature.

It is the primary and essential object of the invention to develop the immunosorbent which would enable to carry out the epilepsy diagnosis and the risk group identification and exhibit specificity, stability and ease of application.

According to the invention said object is accomplished by the provision of a trypsin digest fragment of the glutamate-binding membrane protein from bovine brain tissue having a molecular weight of 5 to 14 kilodaltons, which is bound covalently or ionically to a support material, whereby the preservation of its immunogenic properties is ensured. It is preferred that the support material is polystyrene, which is aminated and subsequently derivatized with glutaraldehyde, or nitrocellulose.

The proposed immunosorbent enables the rapid and effective diagnosis of the epilepsy in a substantial number of patients (up to 200 individuals) simultaneously. Additionally the proposed immunosorbent enables to carry out the prophylactic population screening for the risk group determination and the professional aptitude test with a view to evaluate risk groups among personnel expected to operate under stressing conditions.

Application of the proposed immunosorbent for the epilepsy diagnosis is based on the finding of increased autoantibodies to the mammalian glutamate-binding membrane protein level in the epileptic patient's blood.

The immunogenic fragment of the glutamate-binding membrane protein, bound to a rigid support material, specifically reveals the autoantibodies to glutamate-binding membrane protein in the blood samples of epileptic patients and the individuals involved in the risk group. A diagnosis "epilepsy" is made on the base of two- to four-fold excess of autoantibodies' concentration above control level. Response of the immunosorbent to the blood of patients with other nervous system diseases do not essentially differ from the control level.

The proposed immunosorbent was tested on the laboratory epilepsy model - Krushinsky-Molodkina rats with the inherent predisposition to audiogenic seizures. Normal Wistar rats were used as a control.

The rats were exposed to the acoustical treatment (96 DB), which caused the complete audiogenic seizure pattern. The blood was sampled from the tail vein, frozen and stored at -60°C until use.

Presence of autoantibodies to the glutamate-binding membrane protein was detected with the help of the proposed immunosorbent (immunogenic fragment of the glutamate-binding membrane protein having a molecular weight of 5 to 14 kilodaltons, bound to the polystyrene support material) using the established solid-phase immuno-enzyme analysis protocol. The experiments reveal that the glutamate-binding membrane protein binding in the blood of Krushinsky-Molodkina rats was elevated above the control values. The effect also appeared in the blood of rats not exposed to sound. After the acoustical treatment the glutamate-binding membrane protein binding in the blood of Krushinsky-Molodkina rats significantly increased reaching the maximum level on the 12th - 14th day after the first seizures.

The data obtained provide the evidence of the relationship between the inheritable predisposition to audiogenic seizures and the blood level of anti-glutamate-binding membrane protein autoantibodies. The autoantibodies detected in the blood of Krushinsky-Molodkina rats promote the audiogenic seizure which in turn cause further autoantibodies level elevation.

The proposed immunosorbent was tested in clinic on the blood samples of more than 1000 patients with epilepsy (650 patients), epileptiform syndrome of unknown etiology (187), Parkinson's disease (148) und other neurologic diseases, including schizophrenia (47), Alzheimer's disease (14), as well as 2150 healthy volunteers.

For the investigation the patients' and healthy controls' serum samples were applied in the microtiter wells of the proposed immunosorbent which is the fragment of the glutamate-binding membrane protein from the mammalian brain having a molecular weight of 5 to 14 kilodaltons bound covalently to polystyrene, treated with glutaraldehyde. As a positive control the rabbit polyclonal antiserum against the glutamate-binding membrane protein (titer 1:36000), applied in the microtiter wells in 1:5000 dilution simultaneously with the patients' blood samples, was used. The basal (zero) level was adjusted according to the analysis data of donor serum depleted by the immunoprecipitation with rat brain membrane suspension. After the sample application the microtiter plates were incubated 1 hour at 37°C or at 4°C overnight and binding with the immunogenic fragment of the glutamate-binding membrane protein was determined by the established solid-phase immuno-enzyme analysis protocol using Protein A conjugated with horseradish peroxidase. The results were read with the help of a multichannel spectrophotometer at a wavelength of 492 nm. The results of the analysis were presented in the form of conventional units (CU) : CU = 100 x E₄₉₂, where E₄₉₂ is the extinction at 492 nm.

In another experiment series total (unfractionated) synaptic membrane solubilizate as well as the solubilizates of liver, heart and kidney membranes and the opiate-binding membrane protein, purified from the brain, were used as antigens.

The increased level of autoantibodies to the glutamate-binding membrane protein was observed in the blood of patients with epilepsy and epileptiform syndrome of unknown etiology. In these cases the analysis revealed 89 - 90% of patients. The binding to the immunosorbent of the blood of patients with Parkinson's disease and other central nervous system disorders did not differ from the control level. Other antigens exhibited substantially less specificity. The epileptic patients' recognition level by total synaptic membranes solubilizate was about 60% of the value obtained with the glutamate-binding membrane protein, and by the liver, heart and kidney membranes solubilizates - 32, 36 and 21 % respectively. The purified opiate-binding membrane protein did not virtually bind the components of epileptic sera.

Correlations between the level of autoantibodies to the glutamate-binding membrane protein and the epilepsy dynamics, type and duration were studied in a group of 246 epileptic patients (16 - 50 years old). All patients were subdivided into 3 groups according to the analysis results: I group - 120 - 200, II group - 220 - 320 and III group - above 320 % of the mean donor value. Table 1 presents the group distribution of patients depending on the seizures frequency. The minimal increase of the binding level was detected in the patients with sporadic (up to 1 a year) seizures, the maximal - in the cases of daily or serial seizures.

**Table 1**

| Group Distribution of Epileptic Patients Depending on the Seizures Frequency | | | | |
|---|---|---|---|---|
| N | Seizures frequency | Group distribution (in %) | | |
| | | I | II | III |
| 1 | Sporadic | 83.0 | 11.7 | 5.3 |
| 2 | 4 - 28 a month | - | 81.3 | 18.7 |
| 3 | everyday or serial | - | 11.8 | 88.2 |

Analysis of the autoantibodies titer dynamics demonstrated its gradual increase during the interval between two successive seizures. The substantial effect could be observed on the 8th - 10th day before the seizures, and maximal level was formed immediately after the seizures; it gradually decreased in the subsequent period and reached the initial level in 1 - 2 months after the seizures. The results are presented in the Tables 2 and 3. The data shown in Table 2 illustrate the distinct correlations between the autoantibodies to the glutamate-binding membrane protein level and the type of seizures manifestation. The majority of patients with general type of seizures belonged to II and III groups whereas the patients with fragmentary seizures had low autoantibodies levels (I and II groups). All examined patients with mixed and polymorphous seizures belonged to the III group. In the same group the patients with the epilepsy of unkown etiology were also found. High autoantibodies level was found in patients with inherited type of the disease (Table 3).

**Table 2**

| Group Distribution of Epileptic Patients Depending on the Seizures Type | | | | |
|---|---|---|---|---|
| N | Seizures type | Group distribution (in %) | | |
| | | I | II | III |
| 1 | General | 17.5 | 56.5 | 26.0 |
| 2 | Fragmentary | 62.5 | 25.0 | 12.5 |
| 3 | Mixed and polymorphous | - | - | 100.0 |

**Table 3**

| Group Distribution of Epileptic Patients Depending on Etiology of the Disease | | | | |
|---|---|---|---|---|
| N | Etiology of the disease | Group distribution (in %) | | |
| | | I | II | III |
| 1 | Inherited | - | 50.0 | 50.0 |
| 2 | Posttraumatic | 33.4 | 53.3 | 13.3 |
| 3 | Neuroinfectional | 53.3 | 26.7 | 20.0 |
| 4 | Unknown etiology | 8.3 | 8.3 | 83.4 |

Thus the applicability of the proposed immunosorbent is apparent. The proposed immunosorbent would enable the rapid and effective diagnosis of the epilepsy in a substantial number of patients (up to 200 individuals) simultaneously and to carry out the prophylactic population screening for the risk group determination and the professional aptitude test with a view to evaluate risk groups among personnel expected to operate under stressing conditions. Clinical application of the proposed immunosorbent would allow to improve the patients' status monitoring and optimize the treatment process.

The proposed immunosorbent is produced as follows. The fragment of the glutamate-binding membrane protein from the mammalian brain having a molecular weight of 5 to 14 kilodaltons is bound covalently or ionically to the support material which ensures the preservation of its immunogenic properties. As a support material polystyrene, chromatography paper, nitrocellulose and other materials can be applied.

When the polystyrene is used the polystyrene microtiter plate is treated by nitration to form free nitro groups on the plate surface. The nitro groups are reduced to aminogroups and activated by glutaraldehyde which serves as a linking factor. For the chemical fixation of the immunogenic fragment of the glutamate-binding membrane protein the activated plates are incubated with the previously isolated fragment for 16 hours at 4°C. After the non-bound active groups reduction by 0.1% (weight/volume) of sodium borohydride and washing the wells with 0.9% (weight/volume) of sodium chloride the plate is incubated with a preservative, dried under vacuum and hermetically sealed. After the preservation microtiter plates with the linked immunogenic fragment of the glutamate-binding membrane protein are stored at 4°C.

It is also possible to produce the immunosorbent by binding the immunogenic fragment of the glutamate-binding membrane protein to nitrocellulose strips by the ionic interaction. To this effect an aqueous solution containing the immunogenic fragment of the glutamate-binding membrane protein is applied to the strips and incubated 15 min. Unbound nitrocellulose active groups are blocked by 0.5% (weight/volume) serum albumin with 0.05% (volume/volume) detergent Tween 20. Dried strips are stored up to 1 year in dry place.

Thus fixation of the immunogenic fragment of the glutamate-binding membrane protein achieved in both modes - on the microtiter plate or nitrocellulose strips - prevents the active conformation loss and ensures the preservation of its immunogenic properties.

To promote understanding of the present invention given below are the following examples.

### Example 1

The glutamate-binding membrane protein is isolated from bovine cerebral cortex. Cortex portions (140 g) are homogenized in 500 ml of ice-cold 0.32 M sucrose solution containing phenylmethylsulfonyl fluoride (0.1 mM) and centrifuged at 800 g for 10 min. Supernatant is filtered through 4 layers of nylon gauze and centrifuged at 20000 g for 30 min. The resulting pellet is resuspended in 500 ml of ice-cold distilled water and centrifuged at 8000 g for 30 min. The procedure is repeated twice and the supernatant together with the pellet soft layer is centrifuged at 48000 g 30 min. The resulting fraction (synaptic membranes) is suspended in 1% (weight/volume) solution of sodium deoxycholate in 30 mM Tris-HCl, pH 7.4 and stirred 1 hour at 4°C. The solubilizate is cleared by centrifugation (100000 g for 1.5 hour) and mixed with glutamate-Sepharose 4B (Pharmacia, Sweden), previously suspended in 30 mM Tris-HCl (pH 7.4). The protein binding to the sorbent lasts 16 hours at 4°C. After washing with 6 volumes of buffer, containing 0.5% sodium deoxycholate (weight/volume), the bound glutamate-binding membrane protein is eluted by 1.5 M sodium chloride solution in the same buffer and concentrated by ultrafiltration. The glutamate-binding membrane protein output accounts for about 2 µg/g of brain tissue.

To obtain the immunogenic fragment of the glutamate-binding membrane protein 200 pg of the purified protein is incubated in 20 ml of trypsin solution ("Sigma", USA, type III, 1 mg/mg of glutamate-binding membrane protein) for 3 hours at 37°C. The reaction is terminated by the equivalent quantity of trypsin inhibitor ("Sigma", USA) and the reaction mixture is applied on the Ultrasphere C-18 column (4.6 x 250 mm) for high-performance liquid chromatography. The elution is run with the linear acetonitrile gradient (20 - 68%) at a speed of 1.0 ml/min. Fractions are collected regarding their UV-extinction and subjected to immunoenzyme analysis with the anti-glutamate-binding membrane protein monoclonal antibodies to reveal the presence of the glutamate-binding membrane protein-like immunoreactivity. The immunogenic fragment of the glutamate-binding membrane protein-containing fraction is lyophilized and used for further analysis.

Isolated in such manner the immunogenic fragment of the glutamate-binding membrane protein is characterized biochemically, as the electrophoretically homogeneous polypeptide having a molecular weight of 5 to 14 kilodaltons, and immunologically according to its binding to monoclonal and polyclonal antibodies against the glutamate-binding membrane protein and negative reaction with control antisera against other proteins (interferon and alkaline phosphatase).

For the proposed immunosorbent production wells of the 96-well polystyrene microtiter plate are loaded with a mixture of concentrated sulphuric and nitric acid (2:1), and the plate is incubated at 4°C. After washing by water the wells are filled with tin chloride in concentrated hydrochloric acid (11 g of the salt is dissolved in 10 ml of the acid). The nitro groups reduction lasts for 1 hour at room temperature. Thereafter the plate is thoroughly washed and the wells are loaded with glutaraldehyde (2.5%, weight/volume) in 0.1 M phosphate buffer, containing 0.9% (weight/volume) sodium chloride, and incubated 3 hours at 50°C. After washing the immunogenic fragment of the glutamate-binding membrane protein (0.1 µg in 0.1 ml) is added in each well. The protein binding to the plate continues for 16 hours at 4°C. Aldehyde groups not involved in the reaction are reduced with fresh-prepared sodium borohydride 0,1% (weight/ volume) solution for 30 min at room temperature. After that the wells are washed with the same buffer and loaded with the preservative: mixture of 5% (weight/volume) sucrose and 1% (weight/volume) bovine serum albumin. After the incubation for 1.5 hours at room temperature the plate is vacuum-dried.

The immunosorbent is produced which is in fact the fragment of the glutamate-binding membrane protein having a molecular weight of 5 to 14 kilodaltons bound covalently to the polystyrene microtiter plate.

Stability of the immunosorbent produced was tested by immunoenzyme analysis using monoclonal antibodies and the polyclonal rabbit antiserum against the glutamate-binding membrane protein. The reaction level was stable for 12 months.

Produced immunosorbent is stored at 4°C for 12 months.

### Example 2

The immunogenic fragment of the glutamate-binding membrane protein is isolated after trypsin incubation and chromatography as described in Example 1. The nitrocellulose sheet (80 x 120 mm²) is inserted in the apparatus for dot-blotting and the wells are filled with the purified glutamate-binding membrane protein-solution. After incubation for 15 min the excess solution is removed by vacuum-filtration, and the nitrocellulose sheets are transferred in an aqueous medium containing 0.5% (weight/volume) Tween 20. After incubation at room temperature for 1 hour the sheets are dried under vacuum.

The immunosorbent produced is in fact the immunogenic fragment of the glutamate-binding membrane protein having a molecular weight of 5 to 14 kilodaltons bound ionically to the nitrocellulose. It is stored in a dry place at room temperature for 12 months.

The proposed immunosorbent can be used in the medical practice for the epilepsy diagnosis and the risk group identification.

## Claims

1. Immunosorbent for epilepsy and epilepsy predisposition identification, characterized in that it is the trypsin digest fragment of the glutamate-binding membrane protein from bovine brain tissue having a molecular weight of 5 to 14 kilodaltons, which is bound covalently or ionically to a support material, whereby the preservation of its immunogenic properties is ensured.

2. Immunosorbent according to claim 1, characterized in that the support material is polystyrene, which is aminated and subsequently derivatized with glutaraldehyde, or nitrocellulose.

## Patentansprüche

1. Immunosorbens für die Erkennung von Epilepsie und eine Prädisposition für Epilepsie, dadurch **gekennzeichnet,** daß es das Trypsinverdauungsfragment des glutamatbindenden Membranproteins aus Rinderhirngewebe mit einem Molekulargewicht von 5 bis 14 Kilodalton darstellt, das kovalent oder ionisch an einen Trägerstoff gebunden ist, wodurch die Aufrechterhaltung seiner immunogenen Eigenschaften gewährleistet wird.

2. Immunosorbens nach Anspruch 1, dadurch **gekennzeichnet,** daß der Trägerstoff aminiertes und nachfolgend mit Glutaraldehyd deriviertes Polystyrol oder Nitrozellulose ist.

## Revendications

1. Sorbant immunologique pour l'identification de l'épilepsie et d'une prédisposition à l'épilepsie, ***caractérisé en ce que*** c'est le fragment digéré par la trypsine de la protéine de membrane de liaison du glutamate provenant du tissu cérébral de bovins, d'un poids moléculaire de 5 à 14 kilodaltons, qui est lié de manière covalente ou ionique à un matériau-support, ce qui assure la conservation de ses propriétés immunogéniques.

2. Sorbant immunologique selon la Revendication 1, ***caractérisé en ce que*** le matériausupport est le polystyrène, qui est aminé puis transformé en dérivé avec du glutaraldéhyde, ou la nitrocellulose.
